(19) Europäisches Patentamt European Patent Office Office européen des brevets

(11) **EP 3 701 932 A1**

(12) **EUROPEAN PATENT APPLICATION**

(43) Date of publication:
**02.09.2020 Bulletin 2020/36**

(21) Application number: **19159467.0**

(22) Date of filing: **26.02.2019**

(51) Int Cl.:
**A61K 8/26** *(2006.01)*  **A61Q 11/02** *(2006.01)*
**A61K 8/19** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Unilever PLC**
**London Greater London EC4Y 0DY (GB)**

(72) Inventors:
• **BARILI, Matteo**
**Casalpusterlengo, Lodi 26841 (IT)**

• **GIANNETTO, Francesca**
**Wirral, Merseyside, CH63 3JW (GB)**
• **GUOLI, Angelica**
**Casalpusterlengo, Lodi 26841 (IT)**
• **JOINER, Andrew**
**Wirral, Merseyside, CH63 3JW (GB)**

(74) Representative: **Tansley, Sally Elizabeth**
**Unilever PLC**
**Unilever Patent Group**
**Colworth House**
**Sharnbrook**
**Bedford**
**Bedfordshire MK44 1LQ (GB)**

(54) **ODOUR REDUCING COMPOSITIONS**

(57) Use of a combination of clay and charcoal for inhibiting the release of odorous volatile compounds.

EP 3 701 932 A1

**Description**

The present application relates to compounds that inhibit the odour of volatile compounds, in particular the application relates to compounds that inhibit the odour of volatile compounds in the mouth.

**Field of the Invention**

The present invention is concerned with compounds that inhibit the odour of oral care compositions. More particularly, the present invention is concerned with oral care compositions.

Oral care compositions that freshen the breath have been on the commercial market for some time. Such compositions are usually formulated with perfumes and flavours that mask any odour. Oral care compositions also may comprise surfactants that help clean the mouth.

The present invention has found that certain ingredients can be used to prevent the release of volatile compounds, rather than merely masking.

**Description of the Invention**

The present invention relates to use of a combination of clay and charcoal for inhibiting the release of odorous volatile compounds.

**Detailed Description of the Invention**

Compositions for use in the invention comprise charcoal. Preferably the charcoal has a total surface area (BET) of $900m^2/g$ to $1500m^2/g$ more preferably from $1100 m^2/g$ to 1300 It is also preferred if the charcoal has a density from $250 kg/m^3$ to $370kg/m^3$, more preferably from $300 kg/m^3$ to $340 kg/m^3$. Preferably the charcoal has a D50 particle size from 2 microns to 40 microns, more preferably 5 to 30 microns.

The level of charcoal if used in in oral care composition is preferably from 0.01 to 0.2 wt% of the total composition, more preferably from 0.02 to 0.01 wt% most preferably from 0.03 to 0.08 wt% of the total composition.

Compositions for use in the invention comprise clay, preferably this is a 1:1 layered silicate clay. Typically, the 1:1 layered silicate clay are non-swelling clays. 1:1 layered silicate clay includes kaolinite-serpentine group of clay. Kaolinite-serpentine group of clay includes subgroups of kaolinites and serpentines minerals. Serpentines are trioctahedral sheet minerals which has a tetrahedral sheet and an octahedral sheet having magnesium with minor amounts of aluminium and the species within this subgroup are preferably chrysotile, lizardite and antigorite.

Preferably the 1:1 layered silicate clay is a kaolinites clay. Kaolinites have a dioctahedral sheet and the species included within the kaolinites subgroup are kaolinite, dickite, nacrite and halloysite clay minerals, but not limited thereto. Kaolinite is particularly preferred in the disclosed invention. Kaolinite, commonly known as kaolin clay, is a naturally occurring mineral. Kaolinite may be a calcined kaolin, highly purified calcined kaolin, colloidal kaolin, or hydrated kaolin, but not limited thereto. Preferred kaolinite have particle size distribution such that at least 98 wt percent of the particles have a particle size of 2 microns or less.

Preferably the kaolinite is a refined kaolin and further preferably the refined kaolin includes 38 wt percent Al2O3 and 45wt percent S1O2 and a maximum of 0.5 wt percent of Fe2C>3.

It is especially preferred if the clay is a hydrated aluminium silicate, with less than 0.025% of 53 micron or greater residues.

Preferably the weight ratio of charcoal to clay is from 1:2.5 to 1:700, more preferably from 1:5 to 1:500, most preferably from 1:10 to 1:200

The oral care compositions of the invention may comprise other components in accordance with their nature.

Preferably, the oral care composition comprises water, thickener, surfactant, and abrasive. Suitable thickeners include silicas and calcium carbonate. Suitable surfactants include the alkali-metal alkyl sulphate surfactants such as the sodium alkyl sulphates, the most preferred being sodium laurylsulphate.

Preferred abrasive materials include silicas, aluminas, calcium carbonates, dicalcium phosphates, calcium pyrophosphates, hydroxyapatites, trimetaphosphates, insoluble hexametaphosphates and so on, including agglomerated particulate abrasive materials, usually in amounts between 3 and 60% by weight of the oral care composition. The most preferred abrasives are calcium carbonate due to its ability to ability to inhibit the release of volatile materials.

The oral care compositions according to the invention may comprise further ingredients which are common in the art, such as:

antimicrobial agents, e.g. Triclosan, chlorhexidine, copper, zinc, and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);anti-inflammatory agents such as ibuprofen, flurbiprofen, aspirin, indomethacin etc.; anti-caries agents such as sodium- and stannous fluoride, aminefluorides, sodium monofluorophosphate, sodium trimeta phosphate and casein;plaque buffers such as urea, calcium lactate, calcium glycerophosphate and strontium polyacrylates; vitamins such as Vitamins A, C and E; plant extracts; desensitising agents, e.g. potassium citrate, potassium chloride, potassium tartrate, potassium bicarbonate, potassium oxalate, potassium nitrate and strontium salts;

anti-calculus agents, e.g. alkali-metal pyrophosphates, hypophosphite-containing polymers, organic phosphonates and phosphocitrates etc.;biomolecules, e.g. bacteriocins, antibodies, enzymes, etc.;flavours, e.g. peppermint and spearmint oils; proteinaceous materials such as collagen; preservatives; opacifying agents; colouring agents; pH-adjusting agents;sweeting agents; pharmaceutically acceptable carriers, e.g. starch, sucrose, water or water/alcohol systems etc.;humectants such as glycerol, sorbitol, propyleneglycol, xylitol, lactitol etc.;binders and thickeners such as sodium carboxymethyl-cellulose, xanthan gum, gum arabic etc. as well as synthetic polymers such as polyacrylates and carboxyvinyl polymers such as Carbopol®; buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

The invention will now be illustrated by the following non-limiting Examples. Examples according to the invention are illustrated by a number, comparative Examples by a letter. Amounts are percentages by weight unless otherwise stated. Solutions and fully formulated products were tested.

## Examples

Examples were prepared using the base paste as in Table 1 and post dosing clay and charcoal to give test pastes summarised in Table 2.

**Table 1: Base Paste Formulation**

| Ingredient | Example Aw/w % |
|---|---|
| Water | To 100 |
| Sorbitol | 45.00 |
| PEG | 2.00 |
| Sodium Fluoride | 0.32 |
| Sodium saccharin | 0.25 |
| Gantrez S-97 | 0.052 |
| Silica Thickening | 8.00 |
| Silica Abrasive | 9.00 |
| Cellulose gum | 0.75 |
| Sodium lauryl sulphate | 1.70 |
| Silica granules | 0.30 |

**Table 2: Test formulations summary**

| Formulation |
|---|
| Example A Base Paste |
| Example B Base Paste + 3% Clay |
| Example 1 Base Paste + 3% Clay + 0.01% Charcoal |
| Example 2 Base Paste + 3% Clay + 0.03% Charcoal |

(continued)

| Formulation |
|---|
| Example 3 Base Paste + 3% Clay + 0.05% Charcoal |

Test toothpastes (10g) were mixed with water (40g). The hydrogen sulphide source was produced by dissolving the contents of a supplied capsule in 20mL of water in a sealed vessel. In sequence, 0.5mL of the hydrogen sulfide solution was added to each toothpaste slurry and the jars were then immediately sealed. Each jar was placed on an "up & over" mixer for 7min. Headspace samples were taken using a 1mL syringe and the gaseous samples were then injected into the OralChroma. A water sample was also mixed with the hydrogen sulphide solution and a sample of headspace analysed. The percentage of reduction of hydrogen sulphide ($H_2S$) from baseline for each test toothpaste was calculated using the following formula:

$$\%Reduction = \frac{H_2S \text{ in water} - H_2S \text{ in sample}}{H_2S \text{ in water}} * 100$$

Results for %Reduction of hydrogen sulphide by test toothpastes are shown in Table 3.

Table 3: Mean %Reduction of hydrogen sulphide by test toothpastes

| Formulation | Mean %Reduction | S.E. |
|---|---|---|
| Example A-Base Paste | 50.5 | 3.9 |
| Example - BBase Paste + 3% Clay | 95.4 | 3.1 |
| Example -1 Base Paste + 3% Clay + 0.01% Charcoal | 97.9 | 0.6 |
| Example -2 Base Paste + 3% Clay + 0.03% Charcoal | 97.5 | 0.4 |
| Example 3 -Base Paste + 3% Clay + 0.05% Charcoal | 96.0 | 0.5 |

Results indicate that the addition of clay and charcoal to a toothpaste formulation can enhance the removal of hydrogen sulphide.

A further example is illustrated with Formulation in Table 4 and was compared with a silica control toothpaste formulation not containing clay and charcoal. Results are shown in Table 5.

Table 4: Toothpaste formulations

| Ingredient | Example 4 w/w% | Example w/w % |
|---|---|---|
| Water | To 100 | To 100 |
| Sorbitol | 45.00 | 45.00 |
| PEG | 2.00 | 2.00 |
| Sodium Fluoride | 0.32 | 0.32 |
| Sodium Saccharin | 0.25 | 0.25 |
| Flavour | 1.25 | 1.25 |
| Gantrez | 0.10 | 0.10 |
| Charcoal Powder | 0.05 | - |
| Blue/Green Pigment | 0.19 | 0.19 |
| Sodium Phosphate | 0.03 | 0.03 |
| Thickening silica | 7.29 | 7.29 |
| Abrasive silica | 9.00 | 9.00 |
| Cellulose gum | 0.75 | 0.75 |

(continued)

| Ingredient | Example 4 w/w% | Example w/w % |
|---|---|---|
| Sodium lauryl sulfate | 1.70 | 1.70 |
| Clay | 3.00 | - |
| Silica granules | 0.29 | 0.29 |

**Table 5: Mean %Reduction of hydrogen sulphide**

| Formulation | Mean %Reduction* | S.E. |
|---|---|---|
| Silica control toothpaste | 41.5[A] | 8.8 |
| Silica toothpaste + 3% Clay + 0.05% Charcoal | 99.5[B] | 0.5 |
| *Different letters for Mean %Reduction indicates statistical significance ($p<0.05$) | | |

**Claims**

1. Use of a combination of clay and charcoal for inhibiting the release of odorous volatile compounds.

2. Use according to claim 1 in which the volatile compounds comprise hydrogen sulphide and thiols.

3. Use according to any preceding claim in which the volatile compound is hydrogen sulphide.

4. Use according to any preceding claim in which the clay and charcoal are constituents of an oral care composition.

5. Use according to claim 4 in which the level of charcoal in the total composition if from 0.01 to 0.2 wt% and the level of clay in the total composition is from 0.5 to 7 wt% of clay.

6. Use according to any preceding claim in which the charcoal has a D50 particle size from 2 microns to 40 microns.

7. Use according to any preceding claim in which the clay is kaolin.

8. Use according to any preceding claim in which the weight ratio of charcoal to clay is from 1:2.5 to 1:700.

Europäisches
Patentamt

European
Patent Office

Office européen
des brevets

## EUROPEAN SEARCH REPORT

Application Number

EP 19 15 9467

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 106 137 866 A (NANTONG YUANHANG PHARAMCEUTICAL CHEMICAL CO LTD) 23 November 2016 (2016-11-23) | 1,4,7 | INV. A61K8/26 A61Q11/02 |
| Y | * the whole document * | 2,3,5,6,8 | A61K8/19 |
| X | CN 101 224 179 A (JIAN JIA [CN]) 23 July 2008 (2008-07-23) | 1,4 | |
| Y | * the whole document * | 2,3,5-8 | |
| X | US 2010/158825 A1 (MAESEN THEODORUS [US] ET AL) 24 June 2010 (2010-06-24) | 1,4 | |
| Y | * paragraph [0093]; claims 11,12,20 * * paragraph [0010] * | 2,3,5-8 | |
| Y | JP S63 57532 A (LION CORP) 12 March 1988 (1988-03-12) * the whole document * | 1-8 | |
| Y | JP 2002 145745 A (ISSHIN SANGYO KK) 22 May 2002 (2002-05-22) * the whole document * | 1-8 | TECHNICAL FIELDS SEARCHED (IPC) |
| X | DATABASE GNPD [Online] MINTEL; 11 December 2018 (2018-12-11), anonymous: "Activated Coconut Charcoal Powder", XP055609991, retrieved from www.gnpd.com Database accession no. 6187481 | 1,4 | A61K A61Q |
| Y | * the whole document * | 2,3,5-8 | |

-/--

The present search report has been drawn up for all claims

| Place of search | Date of completion of the search | Examiner |
|---|---|---|
| Munich | 31 July 2019 | Grenouillat, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 19 15 9467

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE GNPD [Online] MINTEL; 13 December 2010 (2010-12-13), anonymous: "Jet Black Whitening Toothpaste", XP055609993, retrieved from www.gnpd.com Database accession no. 1454722 * the whole document * ----- | 1-8 | |

TECHNICAL FIELDS
SEARCHED      (IPC)

The present search report has been drawn up for all claims

| Place of search | Date of completion of the search | Examiner |
|---|---|---|
| Munich | 31 July 2019 | Grenouillat, N |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 15 9467

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2019

| Patent document cited in search report | | Publication date | Patent family member(s) | | Publication date |
|---|---|---|---|---|---|
| CN 106137866 | A | 23-11-2016 | NONE | | |
| CN 101224179 | A | 23-07-2008 | NONE | | |
| US 2010158825 | A1 | 24-06-2010 | JP 2012512872 A | | 07-06-2012 |
| | | | US 2010158825 A1 | | 24-06-2010 |
| | | | WO 2010080267 A1 | | 15-07-2010 |
| JP S6357532 | A | 12-03-1988 | JP 2600653 B2 | | 16-04-1997 |
| | | | JP S6357532 A | | 12-03-1988 |
| JP 2002145745 | A | 22-05-2002 | NONE | | |

EPO FORM P0459

For more details about this annex : see Official Journal of the European Patent Office, No. 12/82